# EUROPEAN PATENT SPECIFICATION

(11) **EP 1 629 090 B1**
(45) Date of publication and mention of the grant of the patent: **05.03.2014**
(21) Application number: 03781869.7
(22) Date of filing: 06.11.2003
(51) Int. Cl.: C12N 5/10, C12N 15/09, C12N 15/63, C12N 15/64, C12N 15/66, C12N 15/67, C12N 15/82, C12N 15/83, C12N 15/87, C12N 15/90

(54) **EXPRESSION OF FOREIGN SEQUENCES IN PLANTS USING TRANS-ACTIVATION SYSTEM**
EXPRESSION VON FREMDSEQUENZEN IN PFLANZEN UNTER VERWENDUNG EINES TRANS-AKTIVIERUNGSSYSTEMS
EXPRESSION DE SEQUENCES ETRANGERES DANS DES VEGETAUX UTILISANT UN SYSTEME DE TRANSACTIVATION

(30) Priority: 06.11.2002 US 424275 P; 25.04.2003 US 465474 P
(43) Date of publication of application: 01.03.2006
(73) Proprietor: iBio, Inc., Newark, DE 19711 (US)
(72) Inventor: YUSIBOV, Vidadi, Havertown, PA 19083 (US); SKARJINSKAIA, Marina, Newark, DE 19711 (US); METT, Vadim, Newark, DE 19711 (US); HULL, Anna, West Growe, PA 19390 (US)
(74) Representative: Cornish, Kristina Victoria Joy
(86) International application number: PCT/US2003/035869
(87) International publication number: WO 2004/044161

(56) References cited:
- WO-A-98/59062
- US-A- 6 077 992
- US-A1- 2004 088 757
- US-B1- 6 395 962
- MORI MASASHI ET AL: "Inducible high-level mRNA amplification system by viral replicase in transgenic plants" PLANT JOURNAL, BLACKWELL SCIENTIFIC PUBLICATIONS, OXFORD, GB, vol. 27, no. 1, July 2001 (2001-07), pages 79-86, XP002286325 ISSN: 0960-7412
- ODELL J T ET AL: "SEED-SPECIFIC GENE ACTIVATION MEDIATED BY THE CRE/LOX SITE-SPECIFIC RECOMBINATION SYSTEM" PLANT PHYSIOLOGY, AMERICAN SOCIETY OF PLANT PHYSIOLOGISTS, ROCKVILLE, MD, US, vol. 106, no. 2, 1 October 1994 (1994-10-01), pages 447-458, XP002912196 ISSN: 0032-0889
- MALLORY ET AL: 'The amplicon-plus system for high-level expression of transgenes in plants' NATURE BIOTECH. vol. 20, June 2002, pages 622 - 625, XP001187595

## Description

This application claims the benefit of U. S. Provisional Application No. 60/424,275 filed November 6,2002 and U. S. Provisional Application No. 60/465,474 filed April 25,2003.

### FIELD OF THE INVENTION

The present invention is directed to the field of protein expression and Molecular Biology where plants are used as units for expression and production of foreign proteins or nucleotide sequences. The new plant-based production system allows controlled expression of foreign sequences in wide range of plants (monocot and dicots) at different growth stages (sprouts, leaf stage, flowering, seed formation and maturation) in different organs (roots, stem, leaves, flowers, seed pods, seed coat, seeds). Specifically, methods are provided for producing transgenic plants with inactive transgenes and activation of the inactive transgenes using viral vectors.

### BACKGROUND OF THE INVENTION

The capability of single plant cell to regenerate and give rise to whole plant with all genetic features of the parent and ii) transfer of foreign genes into a plant genome by a plant- infecting bacterium, *Agrobacterium tumefaciens* (A. *tumenefaciens)* enabled workers in this field to develop new procedures for crop improvement and stable expression of foreign proteins in plants.

In addition to transgenic plants, with the advances made in molecular plant virology, plant viruses have also emerged as promising tools. Plant viruses have features that range from detrimental to potentially beneficial. The substantial crop losses world-wide due to viral infections have prompted the molecular plant virologists to develop genetic systems that allow manipulation of the virus for management of plant diseases. These genetic systems have also led to the use of viruses as tools, since small plus-sense single-stranded RNA viruses that commonly infect higher plants can be used rapidly amplify virus-related RNAs and produce large amounts of protein.

During the last decade, commercial and academic interest in plants as heterologous expression system has significantly increased, especially for the production of biomedically important proteins. Plants have several advantages for producing therapeutic proteins, including lack of contamination with mammalian pathogens; relative ease of genetic manipulation; eukaryotic protein modification machinery; and economical production. Plants might also provide an ideal vehicle for oral delivery of vaccine antigens, since unlike mammalian cells, plant cells are enveloped with a thick cell wall consisting mainly of cellulose and sugars that may provide protection to the antigen inside against degradation in the gastrointestinal tract.

Both transgenic plants and engineered plant viruses have been used in producing foreign proteins in plant. In the early 1990, transgenic plant technology moved to a new arena as a heterologous expression system for antigens from mammalian pathogens. Since then, a variety of medically important antigens have been expressed in transgenic plants, including hepatitis B surface antigen (HBsAg) *E. coli* heat-labile enterotoxin, rabies virus glycoprotein, and Norwalk virus capsid protein.

Plants can also efficiently express and assemble functional immunoglobulins, including , relatively complex secretory IgA (sIgA) molecules composed of four separate polypeptide chains. Again, this points to the potential of transgenic plants in the bioproduction of complex therapeutic proteins. However, technical challenges remain that must be overcome before plant-based production of therapeutic proteins gains widespread acceptance in the commercial arena. Optimization of protein production levels, an important requirement to any heterologous expression system, is one of these challenges. At present, direct expression of recombinant proteins in transgenic plants does not always satisfy the requirement for high levels of protein expression. Plants have an excellent mechanism for turning down or silencing foreign gene expression, particularly when expression is constitutive. It is generally accepted that the duration of foreign gene expression is one of the critical factors that negatively affect accumulation of high quantities of target proteins. The discovery of inducible promoters somewhat circumvents this problem. Thus, there is considerable interest both in basic plant biology and in biotechnological applications, in the use of inducible systems for the expression of target genes introduced into plants. Compared to constitutive promoters, inducible promoters offer numerous advantages and potentials. In particular, inducible promoters allow for the generation of transgenic plants carrying a transgene whose expression at high level is detrimental or even lethal to the host plants. It is also considered that inducible gene expression will be much less sensitive to post-transcriptional gene silencing.

A number of inducible promoters that may allow control over the expression of target genes in transgenic plants have been described. Based on their specificity to a particular class of inducers these promoters could be divided into three groups: i) promoters that are induced at different developmental stages (flowering, senescence, *etc.)* in different organs (roots, flowers, seeds, etc.), ii) promoters that respond to particular environmental signals (heat-shock, nutritional status, pathogen attack or mechanical wounding), iii) promoters that are induced by chemicals of non-plant origin (tetracycline-, glucocorticoid-, ecdysteroid-, copper- and ethanol-inducible promoters). The latter generally utilize non-plant transcription factors that require chemical inducers for activation. Compared to the first two groups of promoters, chemical-inducible systems have much greater potential for a strict temporal and spatial control of the expression of the target gene expression in transgenic plants. Unfortunately, current inducible plant expression systems have some shortcomings, including leaky promoters or commercially unfeasible manufacturing conditions.

An alternative system for the expression of foreign proteins in plants is based on plant virus vectors. Although plant virus vector-based expression systems have a number of advantages (time, efficient engineering and production, level of target protein expression, environmental safety, etc.) compared to that of transgenic plants, they have some limitations as well. For example, the stability and systemic movement of the recombinant virus may be affected by the size of the target gene. Virus-based vectors are probably less applicable in projects that require coordinated expression of multi-subunit proteins, such as antibodies and enzyme complexes.

Accordingly, there is a need for creating better vectors and/or systems that are more suitable for commercial production of proteins in a controlled manner so that it does not create an environmental hazard yet provides for high levels of target protein or polypeptide accumulation in cells regardless of size and complexity of the proteins or polypeptides, the stability of engineered vectors, size and complexity of proteins that can be optimally produced in plants are some of the desirable properties that need to be realized. In addition, the vectors and/or systems must be environmentally friendly and conforming to the regulatory guidelines.

### SUMMARY OF THE INVENTION.

The present invention provides for creating vectors and/or systems that are environmentally friendly and are suitable for commercial production of proteins or polypeptides in a controlled manner regardless of size-and complexity of the proteins or polypeptides. In general aspects the present invention provides a solution by combining environmentally safe transgenic plants and plant viruses to create a transactivation system for production of target proteins in plants.

Specifically, in one aspect of the invention, a transactivation kit for producing a foreign polypeptide of interest in cells of a host plant is provided. It has two components: (i) a transgenic plant and a recombinant viral vector. More specifically, the kit has genetically transformed cells of the host plant having integrated in their nuclear genome, an inactive or silenced foreign nucleic acid sequence, which is capable of encoding, upon its activation, the foreign polypeptide of interest, and a recombinant RNA viral vector capable of infecting the cells of the host plant and encoding therein a recombinase, wherein the inactive or silenced foreign nucleic acid sequence comprises a blocking sequence between the foreign nucleic acid sequence and the promoter (and other regulatory sequences) for driving expression of the foreign nucleic acid sequence, wherein the blocking sequence is flanked on each side by a recombinase target site with a defined 5' to 3' orientation. The recombinant RNA viral vector can have a recombinant genomic component of a class of virus such as TMV (tobamovirus), an alfalfa mosaic virus, an ilarvirus, a cucumovirus or a closterovirus. The recombinant RNA viral vector can be a heterologous viral vector, i. e. , the genomic component has nucleic acid sequences from more than one class of virus. The host plant is a dicotyledon or a monocotyledon.

In an embodiment, the foreign nucleic acid sequence encodes an antigen of interest such as G protein of Respiratory Syncytial Virus, enterotoxin, rabies virus glycoprotein, rabies virus nucleoprotein, hepatitis B surface antigen, Norwalk virus capsid protein, colorectal cancer antigen and gastrointestinal cancer antigen.

In another embodiment, the foreign nucleic acid sequences encode biopharmaceuticals such as erythropoietins, interferons, insulins, monoclonal antibodies, blood factors, Colony Stimulating Factors, Growth Hormones, Interleukins, Growth Factors and Vaccines.

In another aspect of the invention, a method for producing a foreign polypeptide in cells of a host plant is provided. It includes, among other steps, a step of generating a transgenic plant so that nuclear genome of cells of the transgenic plant has a transgenic DNA comprising an inactive or silenced foreign nucleic acid sequence, which is capable of encoding, upon its activation, the foreign polypeptide, a step of infecting the transgenic plant cells with a recombinant RNA viral vector so that it replicates and transiently expresses therein a recombinase; and a step of growing the plant, where the foreign polypeptides are produced in said cells.

### BRIEF DESCRIPTION OF THE DRAWINGS

**Figure 1** shows an embodiment of a transgenic DNA construct (A) containing target gene (TG) that is separated from the promoter by FRT-Stuffer-FRT and a viral 'vector (B) with an FLP activator gene for expression in plants. MP is movement protein.
**Figure 2** shows another embodiment of an Agrobacterial DNA construct (A) that containes target gene (TG) and a viral vector (B) with an activator gene for expression in plants. The TG in this construct is cloned in the background of plant virus sequences to enhance the target expression. The background virus sequences could be a full-length self-replicating molecule (for example tobacco mosaic virus or potato virus X) or molecule that has recognition sequences and can be trans-replicated by the B.
**Figure 3** shows another embodiment of an Agrobacterial DNA construct (A) that contain two target gene (TG 1 and TG2) in tandem that can be activated by: i) FLP recombinase and ii) Gal4 transcription factor and viral vectors that contain activator genes (B and C) FLP recombinase and Gal4 transcription factor, respectively, for expression in plants.

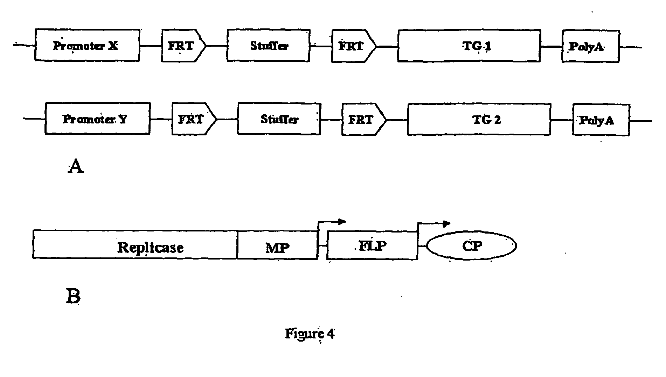

**Figure 4** shows another preferred embodiment of a transgenic DNA construct with target gene (TG) and a viral vector with an activator gene for expression in plants.
**Figure 5** shows another preferred embodiment of a transgenic DNA construct (A) with target genes (TG1 and TG2 each having viral replicon sequences to its upstream end) and viral vectors (B and C) with each with an activator gene for expression in plants.
**Figure 6** shows photographs depicting the expression of GUS in *Nicotiana benthamiana* plants infected with virus vectors producing FLP recombinase. FLP recombinase was cloned into TMV based vectors, AvFLP, D4FLP and D4OASFLP. Here wild type means uninfected control.
**Figure 7** shows photographs depicting the expression of GUS in transgenic plants: FRT⁺/AgoFLP⁻ transgenic plants containing GUS silenced with FRT were agroinfiltrated with agrobacterium containing FLP. FLP⁺/AgroFRT⁻ transgenic plants containing FLP were agroinfiltrated with construct containing FRT. No GUS activity was observed when FRT/FRT or FLP/FLP constructs were used.
**Figure 8** shows photographs depicting the local and systemic expression of GUS in transgenic *Nicotiana benthamiana* plants after inflection with virus vector D4-FLP producing FLP recombinase.
**Figure 9** shows systemic expression of GUS in transgenic N. Benthamiana plants infected with viral vector containing FLP transactivator (FLP recombinase).
**Figure 10** shows a schematic representation of viral vector construct containing GALA-VP16 element. Arrow indicates the site of subgenomic promoter and the wavy line indicates the 3' non-coding region.
**Figure 11** shows a schematic representation of agrobacterial construct containing target gene downstream of DNA binding and activation domains. The inducible system consists of two components; 1, a gene of interest placed downstream of six copies of the GAL4 upstream regulatory sequence (UAS) followed by a minimal promoter (TATA), and 2, a VP16-GAL4 fusion functioning as a transactivator to activate expression of the target gene..
**Figure 12** shows the expression of GUS as a result of trans-activation in plant producing GAL4-VP 16.
**Figure 13A** shows trans-activation of GUS and IA-2 during co-infiltration of *N*. *benthamiana* plants expressing GAL4-VP16.

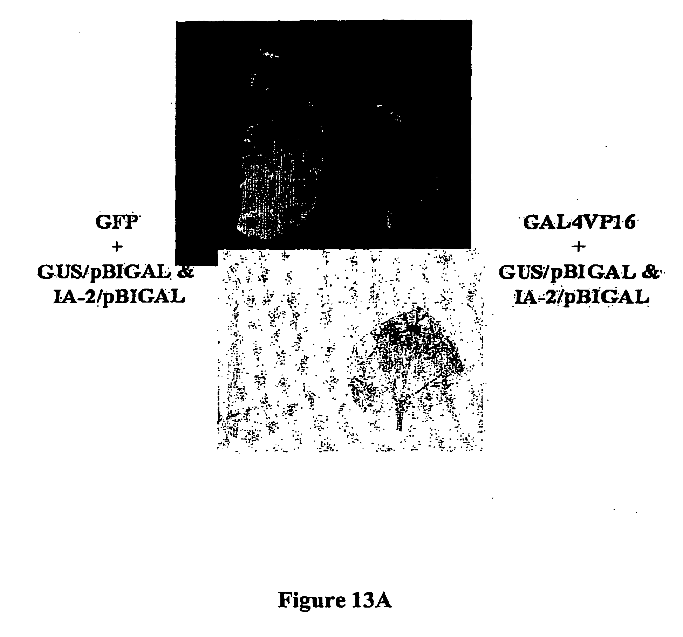

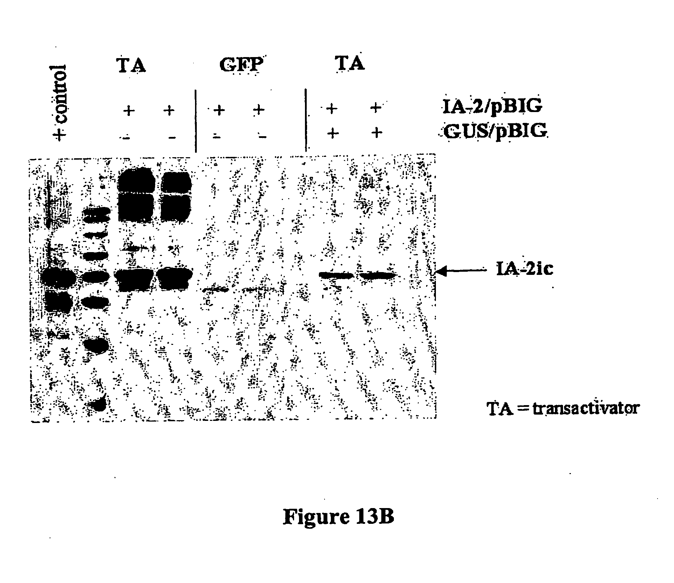

**Figure 13** **B** shows Western analysis of trans-activation of GUS and IA-2ic during Co-infiltration of *N*. *benthamiana* plants expressing GAL4-VP16.

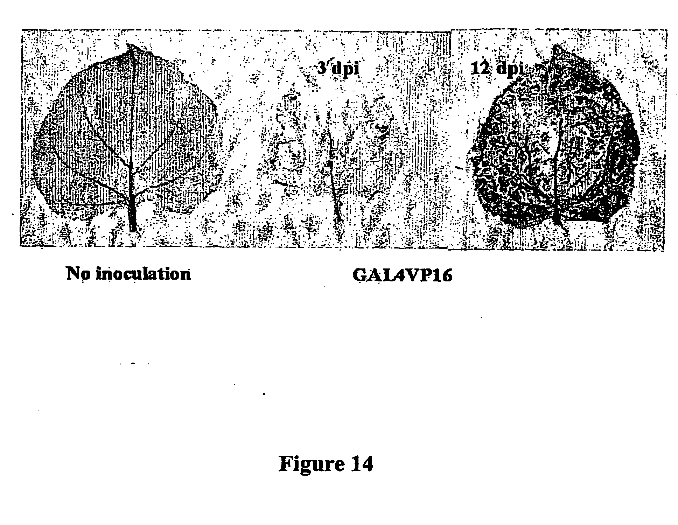

**Figure 14** shows the expression of GUS in pBIGAL/GUS transgenic plants infected with GAL4VP16/D4. The abbreviation"dpi"is days post inoculation.

### DETAILED DESCRIPTION OF THE INVENTION

The present invention provides vectors and methods for expression of foreign sequences (peptides, polypeptides, and RNA) in plants. Specifically, the present invention relates to vectors and methods for activation of silenced or inactive foreign nucleic acid sequence (s) or gene (s) of interest in plant cells for production of peptides, polypeptides, and RNA in such cells. The vectors used for the activation of silenced or inactive sequence (s) are viral vectors.

The activation of silenced or inactive foreign nucleic acid sequence (s) or gene (s) in plant cells is achieved, in trans, by a factor (e. g. , a protein or polypeptide) encoded by a nucleic acid sequence located on the viral vector after the cells are infected with the viral vector. In other words, delivery of activator gene via infection with transient gene delivery viral vector into plant or animal cell activates and results in the expression of target sequence (s). Thus, in the present invention, the activation of silenced or inactive foreign nucleic acid sequence (s) or gene (s) in plant cells is transactivation. It is transactivation because the factor (s) are encoded by nucleic acid sequences that are remotely located, i. e. , on the viral vectors, and the factor (s) are free to migrate or diffuse through the cell to their sites of action.

Accordingly, to practice the present invention, two components are needed as part of the transactivation system. First component of the transactivation system disclosed herein is a genetically transformed cell from a plant of interest containing the foreign nucleic acid sequence (s) or gene (s) of interest. The second component of the transactivation system is a viral vector, preferably a plant viral vector, capable of infecting the genetically transformed cell or organism (i. e. a host cell or a host) and encoding a transacting factor once delivered into the host cell.

To create the first component of the transactivation system, a suitable host cell or a host is genetically transformed with a foreign nucleic acid sequence or a transgene or target gene (TG) of interest to produce a transgenic cell. The genetic transformation is stable, i.e., the transgene is integrated into the host genome and is inheritable from one generation to the next. Plant cells and plants are the host cells or hosts. *Agrobacterium-based* transformation methods may be used to produce transgenic plants containing target gene (s). Several other methods for stable transformation of plants, such as gene gun or vacuum infiltration are known in the art. The terms target gene, transgene and foreign nucleic acid sequence are used interchangeably herein and refer to a nucleic acid sequence introduced into a host cell or a host through a stable genetic transformation procedure, which nucleic acid sequence, if operably linked to a promoter, is capable of encoding a foreign polypeptide or protein of interest such as for example, vaccine antigen, antibodies, therapeutic polypeptides, reporter molecules (e. g., β-glucuronidase (GUS) reporter gene, GFP) etc.

The preferred foreign nucleic acid sequences are those encoding various foreign polypeptides of interest such as, for example, erythropoietins, interferons, insulins, monoclonal antibodies, blood factors, Colony Stimulating Factors, Growth Hormones, Interleukins, Growth Factors, Vaccines, miscellaneous polypeptide drugs such as calcitonin, Leuprolide and HCG etc. These proteins or polypeptides are currently being produced by means other than the transactivation in transgenic cells or organisms described herein and are being marketed under various trade names as biopharmaceuticals.

The target gene of interest may be fused with other sequences that facilitate transport across the cell membranes, tissues and/or systemic delivery. See, for example, U. S. Patent 6, 051, 239 for sequences which can be fused to the target gene of interest. As part of creating the first component of the transactivation system, a nucleic acid construct is introduced into the plant cell or a plant via a genetic transformation procedure. The nucleic acid construct can be a circular construct such as a plasmid construct or a phagemid constuct or cosmid vector or a linear nucleic acid construct including, but not limited to, PCR products. Regardless of the form, the nucleic acid construct introduced is a cassette (also referred to herein as a transfer cassette or an expression cassette) having elements such as promoter (s) and/or enhancer (s) elements besides target gene (s) or the desired coding sequence, among other things. Expression of the target gene, however, depends on transactivation provided by the second component of the invention described further below.

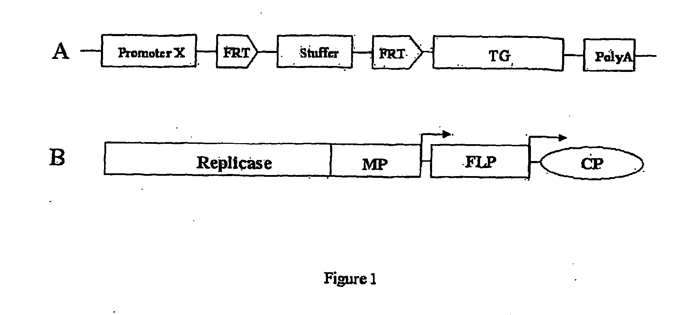

The transactivation system of the invention is a recombinase based transactivation system. A transcription factor type (with activation and binding domains) based transactivation system is also described. In the recombinase based transactivation system, the gene of interest (target gene or TG) is cloned into a transfer cassette (or a transformation plasmid) for integration into the plant genome and stable transformation. The target gene in the transformation plasmid is made non-functional by placing a blocking sequence between the promoter (and other regulatory sequences) for driving the expression of the target gene and the target gene. The resulting transfer cassette (or transgenic DNA) is said to have, among other things, the following structure: promoter-blocking sequence-TG. Figure 1 A shows a schematic representation of a transfer cassette in an embodiment of the present invention.

Promoters used in the present invention can be ubiquitous or constitutive (e. g., Cauliflower Mosaic Virus 35S promoter) or tissue specific promoters (e. g. , potato protease inhibitor II (pin2) gene promoter, promoters from a number of nodule genes). A number of such promoters are known in the art. Inducible promoters that specifically respond to certain chemicals (copper etc. ,) or heat-shock (HSP) are also contemplated. Numerous tissue specific and inducible promoters have been described from plants. The blocking sequence contains a selectable marker element or any other nucleic acid sequence (referred to herein as stuffer) flanked on each side by a recombinase target site (e. g.,"FRT"site) with a defined 5'to 3'orientation. The FRT refers to a nucleic acid sequence at which the product of the FLP gene, i. e. , FLP recombinase, can catalyze the site-specific recombination.

The selectable marker element or stuffer is generally an open reading frame of a gene or alternatively of a length sufficient enough to prevent readthrough. When a suitable recombinase is provided by the second component of the transactivation system to the cells of the transgenic plant containing the transgenic DNA or expression cassette, the recombinase protein can bind to the two target sites on the transgenic DNA, join its two target sequences together and excise the DNA between them so that the target gene is attached to a promoter and/or an enhancer in operable linkage. The recombinase is provided in cells by a viral vector and the recombinase activates the expression of the target gene in cells where it is otherwise silenced or not usually expressed because of the blocking sequence.

It should be noted that the type of recombinase, which is provided to the plant cells in the present invention, would depend upon the recombination target sites in the transgenic DNA (or more specifically in the targeting cassette). For example, if FRT sites are utilized, the FLP recombinase is provided in the plant cells. Similarly, where lox sites are used; the Cre recombinase is provided in the plant cells. If the non-identical sites are used, for example both an FRT and a lox site, then both the FLP and Cre are provided in the plant cells.
The recombinases used herein are sequence-specific recombinases. These are enzymes that recognize and bind to a short nucleic acid site or a target sequence and catalyze the recombination events. A number of sequence-specific recombinases and their corresponding target sequences are known in the art. For example, the FLP recombinase protein and its traget sequence, FRT, are well-characterized and known to one skilled in the art. Briefly, the FLP is a 48 kDa protein encoded by the plasmid of the yeast, *Saccharomyces cerevisiae.* The FLP recombinase function is to amplify the copy number of the plasmid in the yeast. The FLP recombinase mediates site-specific recombination between a pair of nucleotide sequences, FLP Recognition Targets (*FRT's*). The *FRT is a* site for the 48kDa FLP recombinase. The *FRT* site is a three repeated DNA sequences of 13 bp each; two repeats in a direct orientation and one - in an inverted to the other two. The repeats are separated by the 8 bp spacer region that determine the orientation of the FRT recombination site. Depending of the orientation of the *FRT* sites FLP-mediated DNA excision or inversion occurs. FRT and FLP sequences can be either wild type or mutant sequences as long as they retain their ability to interact and catalyze the specific excision. Transposases and integrases and their recognition sequences may also be used.

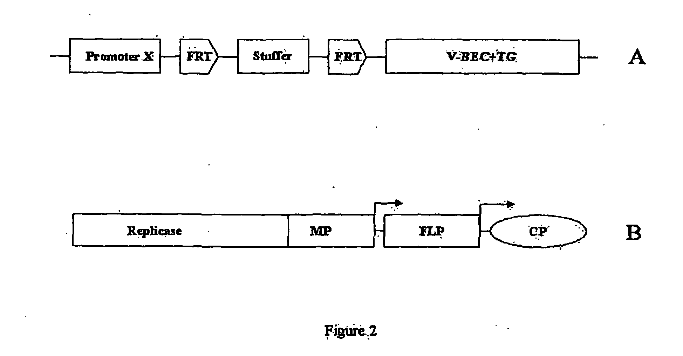

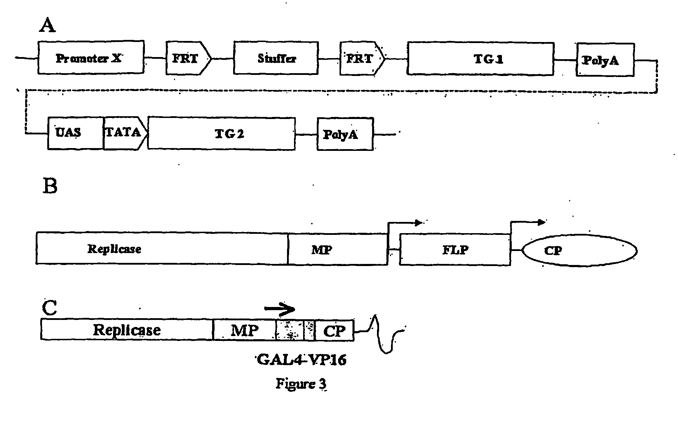

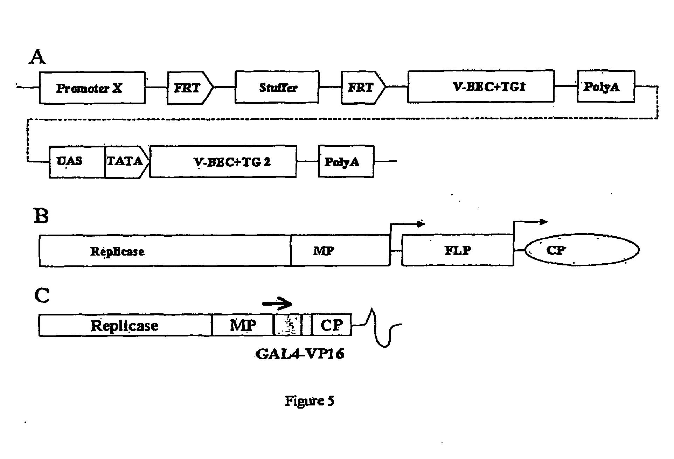

Shown in Figure 2 is a transfer cassette according to another embodiment of the invention. In this embodiment, a viral replicon (e.g., V-BEC) is placed upstream of a target gene (Figure 2A). See also Figure 5A. The viral replicon is a viral nucleic acid sequence which allows for the extrachromosl replication of a nucleic acid construct in a host cell expressing the appropriate replication factors. The replication factor may be provided by a viral vector or a transgenic plant carrying a replicase transgene. Such transgenic plants are known in the art. See, for example, PCT International Publication, WO 00/46350. The constructs of the present invention containing a viral origin of replication, once transcribed, replicate to a high copy number in cells that express the appropriate replication factors. The transfer cassette may contain more than one target gene each linked to a promoter and other elements. Each of the target genes may be transactivated by factors provided by a specific viral vector in a host cell. See, for example, Figures 3 and 5 where it is shown that target genes 1 and 2 are cloned sequentially in a stable transformation vector.

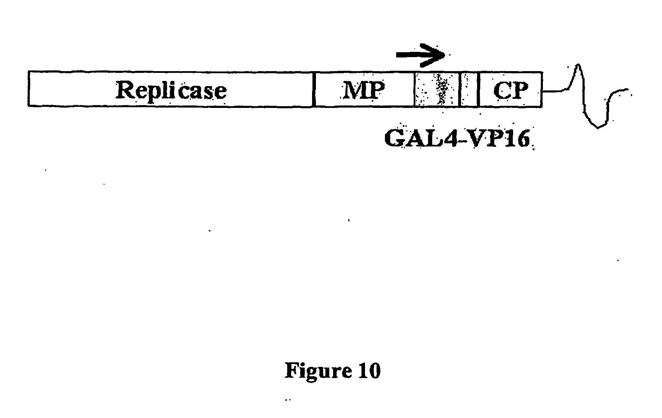

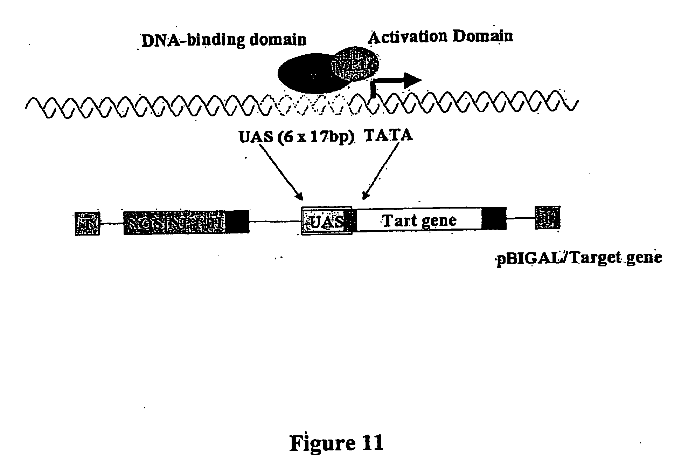

In the transcription factor type (for example with activation and binding domains) based transactivation system, the gene of interest (target gene or TG) is cloned into a transfer cassette (or a transformation plasmid) for integration into the host genome (animal or plant) and stable transformation. The target gene will only be expressed when a suitable transcription factor activity is available. This can happen when a fusion protein containing a DNA-binding domain and an activation domain interacts with certain regulatory sequences cloned into the transfer cassette that is integrated into the host genome. For example, Figures 10 and 11 show constructs for a transcription factor type based transactivation system, specifically the GAL4-V-P16 trans-activation system. It is based on the binding of the yeast GAL4-transcritpion factor to its upstream activating sequence UAS in combination with the strong transcriptional activator VP16 from Herpex Simplex Virus. The viral vector shown in Figure 10 encodes GAL4-VP16 in plants. The transfer cassette shown in Figure 11 has a gene of interest placed downstream of six copies of the GAL4 upstream regulatory sequence (UAS) followed by a minimal promoter (TATA). The GAL4-VP16 fusion protein (as a transactivator) activates the expression of the target gene by binding to the sequences in the UAS.

Specifically, the expression system has: i) a transgene under the control of an artificial promoter containing binding sites for the *Saccharomyces cerevisiae* GAL4 transcription factor and a minimal TATA fragment, and ii) a hybrid GAL4-VP 16 transcription factor (trans-activator) encoded by a virus-based vector. The target gene is not transcribed until the plants are inoculated with the recombinant virus expressing the trans-activator. The trans-gene is placed downstream of six repeats of the yeast GAL4 upstream activating sequence (UAS), followed by a minimal promoter sequence. The transgene activation takes place when a fusion-protein having the GAL4 DNA binding domain and VP16 transcription activating-domain is introduced into the plant via viral-vector mediated transient expression.

There are a number of *trans-acting* factors and the corresponding DNA sequences (i.e., *cis*-acting elements) known in the art. SpI transacting factor and the DNA sequence identical to or a variant of GGGCGG, TFIID transacting factor and the DNA sequence identical to or a variant of TATAAA, C/EBP transacting factor and the DNA sequence identical to or a variant of CCAAT, and ATF-1 transacting factor and the DNA sequence identical to or a variant of GTGACGTA are some examples that can be used in creating the two components of a transactivation system of the present invention.
Viral vectors are used to deliver factors for transactivation of inactive or silenced target genes in transgenic host cells or organisms.

The viral vectors used herein are ofRNA type and do not integrate into host genome and the expression is extrachromosal (transient or in the cytoplasm). Recombinant plant viruses are used in the case of transgenic plant cells or plants. The use of plant viral vectors for expression of recombinases in plants provides a means to have high levels of gene expression within a short time. The autonomously replicating viruses offer several advantages for use as gene delivery vehicles for transient expression of foreign genes, including their characteristic high levels of multiplication and transient gene expression. The recombinant viral vectors used in the present invention are also capable of infecting a suitable host plant and systemically transcribing or expressing foreign sequences or polypeptides in the host plant. Systemic infection or the ability to spread systemically of a virus is an ability of the virus to spread from cell to cell and from infected areas to uninfected distant areas of the infected plant, and to replicate and express in most of the cells of the plant. Thus this ability of plant viruses to spread to the rest of the plant and their rapid replication would aid in delivery of factors for transactivation throughout the plant and the consequent large scale production of polypeptides of interest in a short time.

Therefore, the invention requires construction of recombinant viral vectors by manipulating the genomic component of the wild-type viruses. Preferred viruses are RNA containing plant viruses. Although many plant viruses have RNA genomes, it is well known that organization of genetic information differs among groups. Thus, a virus can be a mono-, bi-, tri-partite virus. "Genome"refers to the total genetic material of the virus. "RNA genome"states that as present in virions (virus particles), the genome is in RNA form..

Some of the viruses which meet this requirement, and are therefore suitable, include Alfalfa Mosaic Virus (AlMV), ilarviruses, cucumoviruses such as Cucumber Green Mottle Mosaic virus (CGMMV), closteroviruses or tobamaviruses (tobacco mosaic virus group) such as Tobacco Mosaic virus (TMV), Tobacco Etch Virus (TEV), Cowpea Mosaic virus (CMV), and viruses from the brome mosaic virus group such as Brome Mosaic virus (BMV), broad bean mottle virus and cowpea chlorotic mottle virus. Additional suitable viruses include Rice Necrosis virus (RNV), and geminiviruses such as tomato golden mosaic virus (TGMV), Cassava latent virus (CLV) and maize streak virus (MSV). Each of these groups of suitable viruses are well characterized and are well known to the skilled artisans in the field. A number of recobminant viral vectors have been used by those skilled in the art to transiently express various polypeptides in plants. See, for example, U. S. Patents 5, 316, 931 and 6,042, 832; and PCT International Publication, WO 00/46350, WO 96/12028 and WO 00/25574. Thus, the methods already known in the art can be used as a guidance to develop recombinant viral vectors of the present invention to deliver transacting factors.

The recombinant viral vector used in the present invention can be heterologous virus vectors. The heterologous virus vectors as referred to herein are those having a recombinant genomic component of a given class of virus (for example TMV) with a movement protein encoding nucleic acid sequence of the given class of virus but coat protein (either a full-length or truncated but functional) nucleic acid sequence of a different class of virus (for example AlMV) in place of the native coat protein nucleic acid sequence of the given class of virus. Likewise, native movement protein nucleic acid sequence instead of the coat protein sequence is replaced by heterologous (i.e. not native) movement protein from another class of virus. Shown in Figures 5B and 5C are schematics of heterologous vectors. For example, a TMV genomic component having an AlMV coat protein is one such heterologous vector. Similarly, an AlMV genomic component having a TMV coat protein is another such heterologous vector. The vectors are designed such that these vectors, upon infection, are capable of replicating in the host cell and transiently activating genes of interest in transgenic plants. Such vectors are known in the art, for example, as described in PCT International Publication, WO 00/46350.

In addition to the genomic elements necessary for infection, replication, movement and spread of the viral vectors, the vectors contain sequences encoding a recombinase (e.g., FLP) or other factor (e.g., GAL4-VP16)

In accordance with the present invention, the host plants included within the scope of the present invention are all species of higher and lower plants of the Plant Kingdom. Mature plants, seedlings, and seeds are included in the scope of the invention. A mature plant includes a plant at any stage in development beyond the seedling. A seedling is a very young, immature plant in the early stages of development. Specifically, plants that can be used as hosts to produce foreign sequences and polypeptides include and are not limited to Angiosperms, Bryophytes such as Hepaticae (liverworts) and Musci (mosses); Pteridophytes such as ferns, horsetails, and lycopods; Gymnosperms such as conifers, cycads, Ginkgo, and Gnetales; and Algae including Chlorophyceae, Phaeophpyceae, Rhodophyceae, Myxophyceae, Xanthophyceae, and Euglenophyceae.

Host plants used for transactivation of genes can be grown either in vivo and/or in vitro depending on the type of the selected plant and the geographic location. It is important that the selected plant is amenable to cultivation under the appropriate field conditions and/or in vitro conditions. The conditions for the growth of the plants are described in various basic books on botany, Agronomy, Taxonomy and Plant Tissue Culture, and are known to a skilled artisan in these fields.

Among angiosperms, the use of crop and/or crop-related members of the families are particularly contemplated. The plant members used in the present methods also include interspecific and/or intergeneric hybrids, mutagenized and/or genetically engineered plants. These families include and not limited to Leguminosae (Fabaceae) including pea, alfalfa, and soybean; Gramineae (Poaceae) including rice, corn, wheat; Solanaceae particularly of the genus *Lycopersicon,* particularly the species *esculentum* (tomato), the genus *Solanum,* particularly the species *tuberosum* (potato) and *melongena* (eggplant), the genus *Capsicum,* particularly the species *annum* (pepper), tobacco, and the like; Umbelliferae, particularly of the genera *Daucus,* particularly the species *carota* (carrot) and *Apium,* particularly the species *graveolens dulce,* (celery) and the like; *Rutaceae,* particularly of the genera *Citrus* (oranges) and the like; Compositae, particularly the genus *Lactuca,* and the species *sativa* (lettuce), and the like and the Family Cruciferae, particularly of the genera *Brassica* and *Sinapis.* Examples of "vegetative" crop members of the family Brassicaceae include, but are not limited to, digenomic tetraploids such as *Brassica juncea* (L.) Czern. (mustard), *B. carinata* Braun (ethopian mustard), and monogenomic diploids such as *B. oleracea* (L.) (cole crops), *B. nigra* (L.) Koch (black mustard), *B. campestris* (L.) (turnip rape) and *Raphanus sativus* (L.) (radish). Examples of "oil-seed" crop members of the family Brassicaceae include, but are not limited to, *B. napus* (L.) (rapeseed), *B. campestris* (L.), *B. juncea* (L.) Czern. and *B. tournifortii* and *Sinapis alba* (L.) (white mustard). Flax plants are also contemplated.

Particularly preferred host plants are those that can be infected by AIMV. For example, it is known in the art that alfalfa mosaic virus has full host range. Other species that are known to be susceptible to the virus are: *Abelmoschus esculentus, Ageratum conyzoides, Amaranthus caudatus, Amaranthus retroflexus, Antirrhinum majus, Apium graveolens, Apium graveolens var. rapaceum, Arachis hypogaea, Astragalus glycyphyllos, Beta vulgaris, Brassica campestris ssp. rapa, Calendula officinalis, Capsicum annuum, Capsicum frutescens, Caryopteris incana, Catharanthus roseus, Celosia argentea, Cheiranthus cheiri, Chenopodium album, Chenopodium amaranticol, Chenopodium murale, Chenopodium quinoa, Cicer arietinum, Cichium endiva, Ciandrum sativum, Crotalaria spectabilis, Cucumis melo, Cucumis sativus, Cucurbita pepo, Cyamopsis tetragonoloba, Daucus carota (var. sativa), Dianthus barbatus, Dianthus caryophyllus, Emilia sagittata, Fagopyrum esculentum, Glycine max, Gomphrena globosa, Helianthus annuus, Lablab purpureus, Lactuca sativa, Lathyrus odatus, Lens culinaris, Linum usitatissimum, Lupinus albus, Lycopersicon esculentum, Macroptilium lathyroides, Malva parvifla, Matthiola incana, Medicago hispida, Medicago sativa, Melilotus albus, Nicotiana bigelovii; Nicotiana clevelandii, Nicotiana debneyi, Nicotiana glutinosa, Nicotiana megalosiphon, Nicotiana rustica, Nicotiana sylvestris, Nicotiana tabacum, Ocimum basilicum, Petunia* × *hybrida, Phaseolus lunatus, Phaseolus vulgaris, Philadelphus, Physalis flidana, Physalis peruviana, Phytolacca americana, Pisum sativum, Solanum demissum, Solanum melongena, Solanum nigrum, Solanum nodiflum, Solanum rostratum, Solanum tuberosum, Sonchus oleraceus, Spinacia oleracea, Stellaria media, Tetragonia tetragonioides, Trifolium dubium, Trifolium hybridum, Trifolium incarnatum, Trifolium pratense, Trifolium repens, Trifolium subterraneum, Tropaeolum majus, Viburnum opulus, Vicia faba, Vigna radiata, Vigna unguiculata, Vigna unguiculata ssp. sesquipedalis,* and *Zinnia elegans.*

An exemplary experimental design for the expression of GFP in transgenic plants through transactivation with recombinase produced in plants via infection with virus can be follows:

A plant virus vector (Av or AlMV) is engineered to express FLP recombinase. The gene for this protein is cloned under subgenomic promoter for coat protein, movement protein or artificial subgenomic promoter. The target gene is cloned into a agrobacterial vector and introduced into nuclear genome to obtain transgenic plants. The target gene is placed under a strong promoter (ubiquitin, dub35, super). However the expression is silenced by the introduction ofNPT or stuffer sequence flanked by FRT (blocking sequence). Target gene is activated by removing the blocking sequences. There can be more than one target gene in a transfer cassette. The target gene(s) is (are) cloned into a agrobacterial vector and introduced into nuclear genome or chloroplast genome. These transformation procedures are well known in the art. Target gene is placed under strong promoter (ubiquitin, dub35, super). However, the expression is silenced by the introduction NPT or stuffer sequences flanked by recombinase recognition sites (e.g, FRT or lox) between the promoter and the TG. The target gene is activated by removing sequences between the promoter and the TG. There could be more than one target gene. The virus vector capable of expressing recombinase in plant cells and transgenic plants (nuclear or chloroplast) that are so made can readily be used to produce target proteins. Transgenic plants are infected with virus containing gene for recombinase.

Inoculation of plants; sprouts, leaves, roots, or stems is done using infectious RNA transcripts, infectious cDNA clones or pregenerated virus material. See, PCT International Publication, WO 00/46350 for guidance on infectious RNA transcripts and procedures for viral infection. Because the time span for target protein production according to the present invention is short (up to 15 days) the expression may not be affected by the gene silencing machinery within the host.

An exemplary experimental design for the GAL4-VP16 trans-activation system is as follows: The GAL4-VP16 fusion protein is expressed from a viral vector. The GAL4-VP16 gene is introduced into a viral vector under the control of a subgenomic promoter for coat protein, movement protein or an artificial subgenomic promoter.
The target gene is cloned into an agrobacterial vector and introduced into the nuclear genome to obtain transgenic plants. The agrobacterial vector can be derived from the vector pBI121 or the like known in the art, in which the 35S CaMV or other promoter is replaced by 6 repeats of the 17 bp GAL4UAS (GCGGGTGACAGCCCTCC) (SEQ ID NO:1) followed by the minimal promoter sequence of 35S CaMV (-46 to +8 bp) or other. Transcription of the target gene can be activated by introduction of the GAL4-VP16 trans-activator.

Once the two components are ready, the transgenic plants can be inoculated with virus carrying the GAL4-VP16 trans-activator to produce the target protein. Inoculation of plants can be made with infectious RNA transcripts, infectious cDNA clones or pregenerated virus material, which are well know in the art.

The transactivation system described herein can be adapted for polypeptide production in transgenic animal cells or non-human organisms (e.g., transgenic mammals, transgenic mouse, transgenic rabbit, transgenic rat, transgenic) containing the target gene of interest. Briefly, a suitable tansformation procedure is used to genetically transform animal cells or organisms. Transfer cassettes can be accordingly designed for transformation by gene gun or electroporation or the like. The viral vectors can be, for example, adenoviral vectors capable of expressing trans acting factors (FLP or GAL4-VP16). Recombinant viral vectors capable of expressing various genes of interest are well known to one skilled in the art. See; for example, U.S. Patents 6,297,357 and 6,596,698.

### EXAMPLES

The following examples further illustrate the present invention. The examples below are carried out using standard techniques, that are well known and routine to those of skill in the art, except where otherwise described in detail. The examples are offered by way of illustration and not by way of limitation.

### Example 1: FLP Recombinase Mediated Transactivation of Inactive Transgenes in Cells:

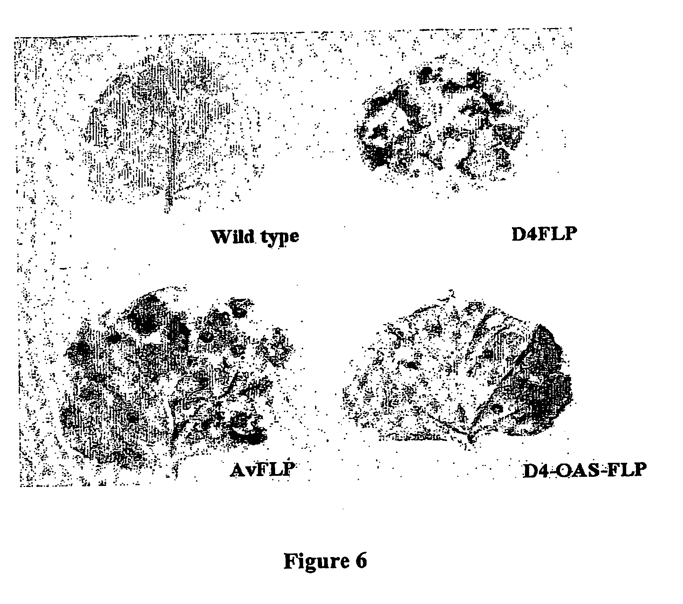

Construction of FLP encoding viral vector and activation of silenced GUS gene in plant cells were carried out as described in the paragraphs below. (For silencing or inactivation of the GUS gene in plant cells a transformation cassette in which the GUS gene was separated from CaMV 35S RNA promoter by the FRT flanked *neo* sequence or the stuffer sequence as schematically shown in Figure 1A was used.
1. Engineering of virus constructs containing FLP recombinase. A open reading frame of yeast recombinase FLP is PCR-amplified from pJFLO plasmid (Luo H, Lyznik LA, Gidoni D, Hodges TK. 2000 FLP-mediated recombination for use in hybrid plant production. Plant J. 23:423-30) using 5'CGC GGA TTC AAT TAA TTA TGC CAC AAT TTG GTA TAT TAT G3' (SEQ ID NO:2) and 5'CCA CTC GAG TTA TAT GCG TCT ATT TAT GTA G3' (SEQ ID NO:3) as 5' and 3' primers, respectively. BamHI and PacI restriction sites at the 5' and XhoI at the 3' end, respectively were introduction for cloning into final vector. Resulting PCR fragment is digested with BamHI and XhoI and cloned in pBluescript. Recombinant plasmid are analyzed by restriction mapping and sequencing to confirm the coding sequences.
2. After sequence confirmation 1.3 kb PacI-XhoI fragment that contained FLP recombinase open reading fragment (from 1) was cloned into a different TMV virus based expression vectors, including D4, Av, and 30B. The insert is confirmed by restriction digest analysis.
3. Plasmid DNA from recombinant virus constructs, containing FLP recombinase was used to synthesize in *vitro* transcripts using T7 RNA polymerase. The resulting RNA transcripts were used to inoculate leaves of reporter transgenic plants transformed with pFFG (Luo H, Lyznik LA, Gidoni D, Hodges TK. 2000 FLP-mediated recombination for use in hybrid plant production. Plant J. 23:423-30) containing GUS gene separated from CaMV 35S RNA promoter by the *neo* sequence flanked by FRT sites.
4. The inoculated leaves from (3) are sampled after a period of growth at different time points and the activity of GUS reporter enzyme is visualized using histochemical staining with X-gluc substrate. Blue staining indicates expression of the reporter as a result of excision of *neo* cassette by FLP recombinase (Fig. 6, 7, 8, and 9).
5. The experiment is conducted as in (3 and 4) except for co-inoculation with in *vitro* transcript from AvA4 providing systemic movement function *in trans.* In this case blue staining in non-inoculated upper leaves indicates systemic phloem movement of the FLP-expressing RNA, which activates the expression of GUS gene as a result of excision of *neo* cassette.

### A: Expression of β-glueuronidase reporter gene in Nicotiana benthamiana plant by trance-activation via infection with virus vector containing FLP recombinase:

To demonstrate that the inducible plant expression system described above allows for tight controlled expression of a trans-gene that is placed downstream of stuffer flanked with FRT sequences the inventors introduced into plants, a viral vector (see Fig 1B) transiently expressing FLP activator and followed with agroinfiltration of construct containing silenced GUS. It is shown here that the target proteins can be expressed in a fully transient manner.

In brief, *Nicotiana benthamiana* plants (at 6 leave stage) were independently inoculated with *in vitro* transcripts of the viral vectors containing FLP (trans-activator) gene under the control of subgenomic promoter for mRNA of viral coat protein. Approximately five to seven days post-inoculation symptoms (curling of leaves) of virus infection could be observed.

Ten days post inoculation with *in vitro* transcripts of the viral vectors containing FLP the leaves were infiltrated with an agrobacterium suspension carrying GUS gene silenced by introducing stuffer flanked with FRT sequences. Strong β-glucuronidase activity was observed in all leaves which expressed the FLP trans-activator and were infiltrated with agrobacterium containing the β-glucuronidase target gene, while no β-glucuronidase activity was detectable in any of the control leaves (Figure 6). In addition, no β-glucuronidase activity was observed in leaves that expressed FLP but did not receive β-glucuronidase gene during agro-infiltration.

This is a strong indication that the FLP/FRT trans-activation system allows for tight controlled expression of the desired target gene. The trans-activation system described in this document can be used for expression of both single subunit as well as multi-subunit complex target proteins, including antibodies that require simultaneous expression of two target genes.

### B: Expresssion of target-gene in transgenic plants by trans-activation via agroinfiltration:.

A. To show that the system allows expression of target gene in transgenic plants, transgenic *N. benthamiana* plants containing either GUS gene silenced by stuffer or FLP recombinase were created.

*Plant Transfonnation: Nicotiana benthamiana* were transformed with pBI-based target constructs, containing target gene bordered with FRT elements or GAL4 transcription activator binding site as follows: The youngest, fully expanded leaf, was sterilized in 20 % bleach containing 0.01% Tween for 7 min and then rinsed three times in sterile water. A 100 ml saturated culture of agrobacterium (strain GLA 4404) was resuspended in 20 ml MS media. The sterilized leaves were incubated in the agrosuspension for 5 to 10 min. During the incubation the leaves were cut into one by one cm pieces. The leaf-pieces were co-cultivated in the dark at 21 °C on MS-2 plates (MS media with 20 g/l sucrose and 0.7% agar) for three days where after they were transferred to regeneration media (MS salts, 30 g/l sucrose, 0.7 % agar, 0.2 mg/l NAA, 1 mg/l BA) containing 1mg/l zeatin, 150 mg/l Km and 500 mg/l cefotaxime. When shoots appeared they were transferred to rooting media (½ MS media containing 20 % sucrose and 0.7 % agar) with 250 mg/l cefotaxime. Plantlets with roots were transferred to soil and acclimated under plastic wrap for 4- 7 days.

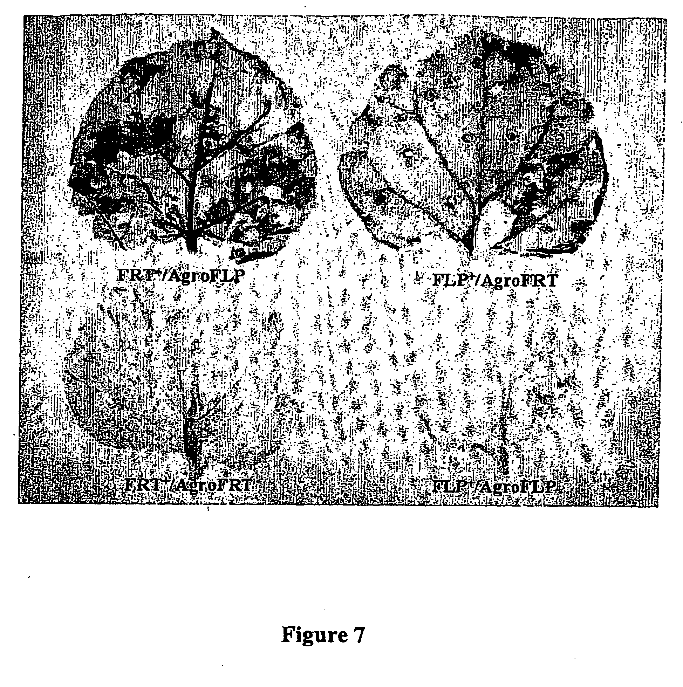

Mature leaves of each of the transgenic plants were used to test the trans-activation. i) Plants transgenic for GUS were infiltrated either with agrobacterium containing transformation vector with FLP recombinase or FRT elements. Expression of GUS was observed only in transgenic plants infiltrated with agrobacterium containing FLP recombinase (Figure 7). When transgenic plants containing GUS gene silenced by FRT elements were infiltrated with agrobacterium carrying GUS gene flanked with FRT sequences no GUS activity was detected. ii) However, when transgenic plants expressing FLP recombinase were infiltrated with agrobacterium carrying GUS gene flanked with FRT sequences or FLP only. GUS activity was detected only when plants were agroinfiltarted with agrobacterium carrying GUS gene flanked with FRT sequences (Figure 7).

### C. Expresssion of target gene in transgenic plants by trans-activation via virus infection:

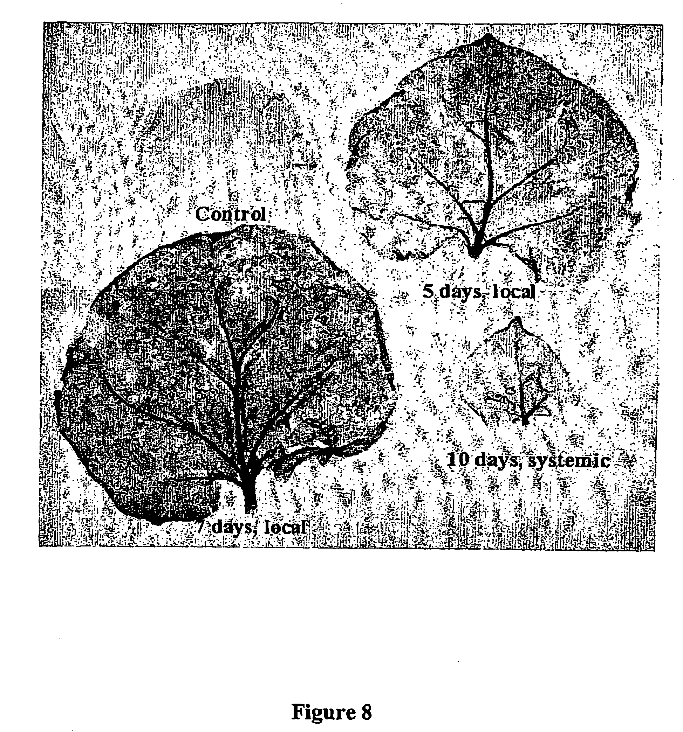

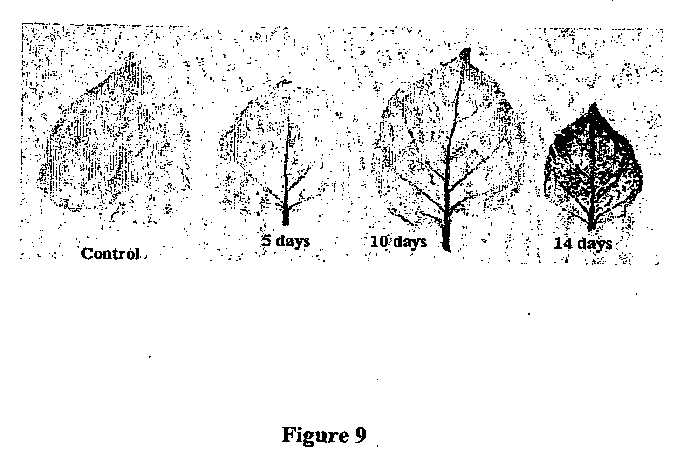

To demonstrate that the system allows for expression of target gene in stably transformed plants, transgenic *N. benthamiana* plants containing GUS gene silenced by stuffer flanked with FRT sequences were created. These transgenic plants were inoculated with virus vector containing FLP recombinase. Infection with virus containing FLP recombinase resulted in significant GUS activity, both locally and systemically (Figure 8 and 9). No β-glucuronidase activity was observed in the absence of the FLP trans-activator (Figure 8, 9,transgenic un-infected control plant). Thus, we have demonstrated that the FLP/FRT system can be used for the controlled expression of target genes in transgenic plants via trans-activation with plant virus-produced FLP recombinase.

### Example 2: GALA-VP16 Mediated Transactivation of Inactive Transgenes in Cells GAL4-VP16-trans-activator system

### DNA constructs:

The vector pET-15 GAL4-VP16 UASmGFP5ER encodes the GAL4-VP16 gene-fusion. GAL4-VP16 was PCR amplified using oligonucleotides GAL4VP16-5' (5'-CCAGGATCCTTAATTAATGAAGCTCCTGTCCTC-3') and GAL4VP16-3' (5'-ACGCGTCGACAGATCTACCCACCGTA-3') to introduce 5' *Pac*I and a 3' *Sal* I cloning sites for cloning into viral vectors based on AlMV, TMV, or CMV (Figure 10).

### GAL4 UAS construction and cloning:

Six copies of the GAL4 UAS was constructed by annealing the two complimentary oligonucleotides 6XGAL4 (5'-GCGGGTGACAGCCCTCCGCGGGTGACAGCCCTCCGCGGGTGACAGCCCTCCG CGGGTGACAGCCCTCCGCGGGTGACAGCCC TCCGCGGGTGACAGCCCTCCGT-3') (SEQ ID NO:4) and Pst6XGAL4Xba (5'-CTAGACGGAGGG CTGTCACCCGCGGAGGGCTGTCACCCGCGGAGGGCTGTCACCCGCGGAGGGC TGTCACCCGCGGAGGGCTGTCACCCGCGGAGGGCTGTCACCCGCTGCA-3'), which create a 5' *Pst* I overhang and a 3'*Xba* I overhang. The GAL4 fragment was cloned into pBluescript for sequence analysis.

Similarly, the minimal -46 to +8 region of the CaMV promoter was constructed by annealing the two complimentary oligonucleotides CaMVXba (5'-CTAGAGCGAAGACCCTTCCTCTATATAAGGAAGTTCATTTCATTTGGA GAGGACACGCTG-3') and CaMVBam (5'-GATCCAGCGTGTCCTCTCCAAATGAA ATGAACTTCCTTATATAGAGGAAGGGTCTTGCT-3'). This DNA fragment was also cloned into pBluescript for sequence analysis.

Following sequence analysis the two promoter components were cloned in tandem into pBluescript as follows. The minimal promoter was excised using *Bam*HI and *Xba*I*.* The 6xGALAUAS was excised with XbaI and *Hind*III*.* The two fragments were ligated into pBluescript simultaneously and analyzed by DNA sequencing. The *Hind*III *- Bam*HI fragment, encompassing both the 6xGAL4UAS and the minimal promoter sequences, was cloned into pBI121 where it replaced the 35SCaMV promoter. In the resulting vector, referred to as pBIGAL (Figure 11), reporter. (β-glucuronidase)-expression is driven by the GAL4 promoter upon binding by the GAL4-VP16 trans-activator. The β-glucuronidase encoding gene can be replaced by other target genes using the 5' restriction site Bam HI and the 3' site *Sac*I*.*
IA-2 was cloned into the 5' *Bam*HI site and 3' *Sac*I sites of pBIGAL.

### Viral infection:

*In vitro* transcripts were synthesized using T7 RNA polymerase after *Kpn*I linearization of TMV-based vector containing either GFP or GAL4-VP16 as follows. Approximately 10 µg of DNA was linearized with 30 units *of KpnI* overnight in a reaction volume of 100 µl. Four µl of the restriction digest was used to produce in vitro transcripts using the AmpliCap T7 High Yield message Maker Kit (Epicentre) according the manufacturers recommendations. Transcripts from one such reaction were used to infect 12 six-week-old *Nicotiana benthamiana* (transgenic or wildtype) by manually applying the transcripts dissolved in FES [sodium-pyrophosphate 1% (w/v), macaloid 1% (w/v), celite 1% (w/v), glycine 0.5 M, K₂HPO₄ 0.3 M, pH 8.5, with phosphoric acid] onto young, fully expanded leaves.

### Agro-infiltration of a single (reporter) gene:

pBIGAL was transformed into *Agrobacterium tumefaciens* strain GLA 4404 and used to infiltrate *N. benthamiana* leaves that had previously been inoculated with virus expression vector containing GAL4-VP16 or GFP. Recombinant virus infection was monitored by observing GFP accumulation under UV-light. Because the GAL4-VP16 infection progression could not be visualized definitely it was inferred that GAL4-VP16 infection would progress similar to that of vector containing GFP progression due to the similarity in insert size of GFP and GAL4VP 16. Agro-infiltration was performed when GFPaccumulation could be visualized. In addition, phenotypic symptoms indicative of viral infection were monitored.

An Agrobacterium culture of pBIGAL was grown to saturation in YEB containing 50 µg/ml Kanamycin (Km) at 28 °C, 225 rpm. This culture was diluted 1:500 into YEB containing 50 µg/ml Km, 10 mM MES and 20 µM acetosyringone and grown at 28 °C, 225 rpm to an OD₆₀₀ of 0.8. The cells were collected by centrifugation at 3000 g for 15 min and resuspended in MMA (1x MS salts, 10 mM MES pH 5.6,20 g/l sucrose, 200µM acetosyringone) to and OD₆₀₀ of 2.4. The MMA suspension was held at room temperature for 1-3 hrs before infiltration. Infiltration with agro was performed by gently injecting the acetosyringone-induced suspension of agrobacterium into virally infected, fully expanded, young leaves so that the agro-suspension diffused through the tissue without damaging the leaf. The leaves were left on the plants for three to four days after agro-infiltration and thereafter harvested, photographed under UV-light (to demonstrate green fluorescence of GFP in controls) and assayed for β-glucuronidase -activity by vacuum-infiltration in GUS-buffer (0.1 m NaPO₄ pH 7.1, 0.5 mM K₃[Fe(CN₆)], 0.5 mM K₄[Fe(CN₆)], 10 mM EDTA, 0.01 % Triton X-100, 1 mg/ml x-gluc) followed by 7 hrs incubation at 37 °C. The plants were de-stained in 70% ethanol over several days and photographed on a light box.

### Agro-infiltra/ion o two target genes β-glucuronidase and IA-2:

pBIGAL carrying either, β-glucuronidase or IA-2, was transformed into *Agrobacterium tumefaciens* strain GLA 4404 and used to infiltrate *N. benthamiana* leaves that had previously been inoculated with a recombinant virus vector containing GAL4-VP16 or GFP as described above. Leaves expressing the GAL4-VP16 trans-activator or the GFP control were infiltrated with either β-glucuronidase/pBIGAL or IA-2/pBIGAL alone or with a mixture of β-glucuronidase/pBIGAL and IA-2/pBIGAL. Three to four days post-agro-infiltration the leaves were harvested and photographed. All the leaves that had been infiltrated with agrobacterium containing the β-glucuronidase/pBIGAL vector were assayed for β-glucuronidase activity as described above. Roughly half the leaves that had been infiltrated with both β-glucuronidase/pBIGAL and IA-2/pBIGAL constructs were assayed for β-glucuronidase activity, while the other half was sampled for Western blot analysis. All IA-2/pBIGAL infiltrated leaves were sampled for Western blot analysis as well. Westerns were performed as follows: Two to three one cm circular leaf-disk were taken from each leaf and frozen at -80 °C min microcentrifuge tubes. The leaf-tissue was homogenized in 100 µl 0.1 M sodium phosphate buffer, pH 7.1 using a micro-pestle attached to a *Sears-brand small drill.* Following homogenization, 50 µl protein SDS gel loading buffer was added to the samples and the samples were heated to 100 °C for 5 min. The samples were centrifuged at full speed in a microcentrifuge for 20 min to collect the plant debris. 10 µl of the supernatant was loaded onto a 10 % polyacrylamide gel and proteins were separated by electrophoresis followed by transfer onto PVDF membrane. The membrane was blocked overnight in I-block and probed with a primary mouse monoclonal anti-tyrosine phosphatase (IA-2ic) antibody (LAD), diluted 1: 1000 in I-block, followed by a secondary horse-radish peroxidase conjugated anti mouse antibody, diluted 1:10,000 in I-block. Westerns were developed with Pierce chemiluminescent detection kit according to the manufacturers directions.

### A. Expression of β-glucuronidase reporter gene in Nicotiana benthamiana plant trans-activation GAL4-VP16:

To demonstrate that the inducible plant expression system described above allows for tight transcriptional control of a trans-gene the β-glucuronidase reporter gene was placed downstream of 6 repeats of the 17 bp GAL4 UAS and introduced into plants transiently expressing the GAL4-VP16 activator by agro infiltration. It is shown here that the target proteins can be expressed in a fully transient manner.

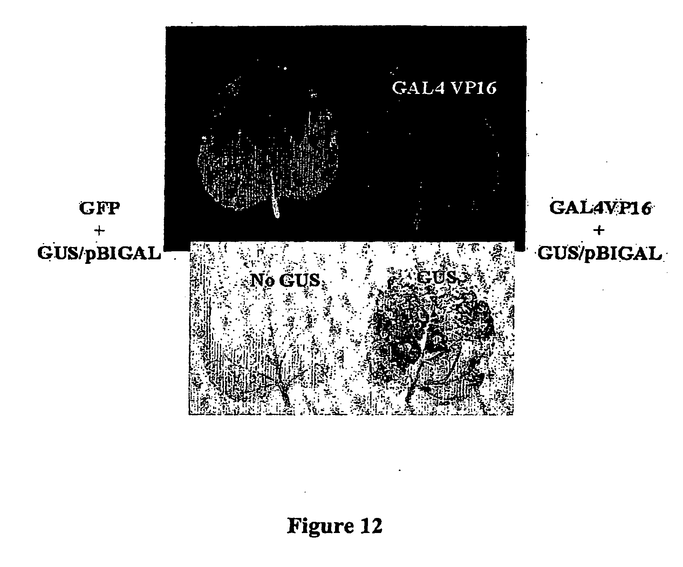

*Nicotiana benthamiana* plants (at 6 leave stage) were independently inoculated with *in vitro* transcripts of the viral vector containing GAL4-VP16 (trans-activator) or GFP (control) gene under the control of subgenomic promoter for mRNA of viral coat protein. Approximately five to seven days post-inoculation symptoms (curling of leaves) of systemic spread of virus infection could be observed. In plants inoculated with viral vector containing GFP expression of GFP could be monitored by visualizing the green fluorescence of GFP under UV-light in upper leaves (Figure 12).

Ten days post inoculation with *in vitro* transcripts of the viral vectors containing GAL4-VP16 trans-activator or GFP gene, the upper leaves were infiltrated with an agrobacterium suspension carrying pBIGAL that contain the target gene, β-glucuronidase. Two to three days post agro-infiltration the leaves were harvested and assayed for β-glucuronidase activity (Figure 12). Strong β-glucuronidase activity was visualized in all leaves which expressed the GAL4-VP16 trans-activator and were infiltrated with agrobacterium containing the β-glucuronidase target gene, while no β-glucuronidase activity was detectable in any of the control leaves, which expressed GFP (Figure 12). In addition, no β-glucuronidase activity was observed in leaves that expressed GAL4-VP1 6 but did not receive β-glucuronidase/pBIGAL during agro-infiltration. Accordingly, the GAL4-VP16 trans-activation system allows for tight controlled expression of the desired target gene.

### B. Expresssion of two target genes:

The trans-activation system described above can be used for expression of both single subunit as well as multi-subunit complex target proteins, including antibodies that require simultaneous expression of two target genes.
To demonstrate that the system allows for expression of more than one target gene, *N*. *benthamiana* plants were infected with a viral vector containing GAL4-VP16 were infiltrated with agrobacterim carrying two target genes that remain inactive unless transactivated by GAL4-VP16 transcription activator (β-glucuronidase and IA-2: Kudva YC, Deng YJ, Govindarajan R, Abraham RS, Marietta EV, Notkins AL, David CS. HLA-DQ8 transgenic and NOD mice recognize different epitopes within the cytoplasmic region of the tyrosine phosphatase-like molecule, IA-2. Hum Immunol. 2001 Oct;62(10):1099-105.). The plants were assayed for β-glucuronidase activity and probed for IA-2 expression at 3 days post agro-infiltration. Simultaneous expression of both β-glucuronidase and IA-2/pBIGAL (Figures 13A and 13B) was observed. No β-glucuronidase activity was observed in the absence of the GAL4-VP16 trans-activator (Figure 13A), neither was there any detectable β-glucuronidase activity in plants that were infiltrated with MMA alone (not shown). Similarly, IA-2 was detected only in plants that expressed the trans-activator and not in plants expressing the GFP control (Figure 13B). Moreover, the level of IA-2 expression was not adversely affected by the presence of a second target gene, β-glucuronidase. Thus, it has been demonstrated here that the GAL4-VP16 trans-activation system can be used for the expression of two target genes in *Nicotiana Benthaniiana.*

### C. Expression of target gene in transgenic plants by trans-activation:

To demonstrate that the system allows for expression of target gene in stably transformed plants transgenic *N. benthamiana* plants containing GUS gene were created. These transgenic plants were inoculated with virus vector containing GAL4-VP16. Three and 12 (Fig. 14) days post inoculation leaves from inoculated and non-inoculated plants were assayed for β-glucuronidase activity. In leaves harvested from GAL4-VP16/D4 inoculated plants expression of (3-glucuronidase was observed (Figure 14). No β-glucuronidase activity was observed in the absence of the GAL4-VP16 trans-activator (Figure 14), neither was there any detectable β-glucuronidase activity in plants that were infiltrated with MMA alone (not shown). Thus, it has been demonstrated here that the GAL4-VP 16 trans-activation system activates the expression of silent target genes in transgenic plants.

## Claims

1. A kit for producing a foreign polypeptide of interest in cells of a host plant comprising:
genetically transformed cells of the host plant having integrated in their nuclear genome, an inactive or silenced foreign nucleic acid sequence encoding a foreign polypeptide of interest; and
a recombinant RNA viral vector capable of infecting the genetically transformed cells of the host plant and encoding therein a recombinase, wherein the inactive or silenced foreign nucleic acid sequence comprises a blocking sequence between the foreign nucleic acid sequence and the promoter (and other regulatory sequences), for driving expression of the foreign nucleic acid sequence wherein the blocking sequence is flanked on each side by a recombinase target site with a defined 5' to 3' orientation.

2. The kit of claim 1, wherein the recombinant RNA viral vector has a recombinant genomic component of a tobamovirus, an alfalfa mosaic virus, an ilarvirus, a cucumovirus or a closterovirus.

3. The kit of claim 1, wherein the host plant is a dicotyledon or a monocotyledon.

4. The kit of claim 1, wherein the foreign nucleic acid sequence comprises a viral sequence that self-replicates in the transformed cells of the host plant following transactivation.

5. The kit of claim 1, wherein the blocking sequence comprises a stuffer sequence flanked on each side by an FRT site in a 5' to 3' orientation.

6. The kit of claim 1, wherein the foreign nucleic acid sequence encodes an antigen.

7. The kit of claim 6, wherein the antigen is selected from the group consisting of G protein of Respiratory Syncytial Virus, enterotoxin, rabies virus glycoprotein, rabies virus nucleoprotein, hepatitis B surface antigen, Norwalk virus capsid protein, colorectal cancer antigen and gastrointestinal cancer antigen.

8. The kit of claim 1, wherein the said cells have one or more types of foreign nucleic acid sequences capable of encoding biopharmaceuticals.

9. The kit of claim 8, wherein the biopharmaceuticals are selected from the group consisting of erythropoietins, interferons, insulins, monoclonal antibodies, blood factors, Colony Stimulating Factors, Growth Hormones, Interleukins, Growth Factors and Vaccines.

10. The kit of claim 1, wherein the recombinant RNA viral vector is a heterologous viral vector.

11. The kit of claim 1, wherein the inactive or silenced foreign nucleic acid sequence is introduced into the host plant using an Agrobacterial DNA construct and in which a viral replicon is placed upstream of a target gene and where the viral replicon is a viral nucleic acid sequence which allows for the extrachromosomal replication of a nucleic acid construct in a host cell expressing the appropriate replication factors.

12. A method for producing a foreign polypeptide in cells of a host plant comprising:
(a) providing a transgenic plant so that nuclear genome of cells of the transgenic plant has a transgenic DNA comprising an inactive or silenced foreign nucleic acid sequence encoding a foreign polypeptide;
(b) infecting the transgenic plant cells with a recombinant RNA viral vector so that it replicates and transiently expresses therein a recombinase; and
(c) growing said plant, wherein said foreign polypeptides are produced in said cells; wherein the inactive or silenced foreign nucleic acid sequence comprises a blocking sequence between the foreign nucleic acid sequence and the promoter (and other regulatory sequences), for driving expression of the foreign nucleic acid sequence wherein the blocking sequence is flanked on each side by a recombinase target site with a defined 5' to 3' orientation.

13. The method of claim 12, wherein the recombinant RNA viral vector is a heterologous viral vector.

14. The method of claim 12, wherein the host plant is a dicotyledon or a monocotyledon.

15. The method of claim 12, wherein the recombinant RNA viral vector has a recombinant genomic component of a tobamovirus, an alfalfa mosaic virus, an ilarvirus, a cucumovirus or a closterovirus.

16. The method of claim 12, wherein the foreign nucleic acid sequence comprises a viral sequence that self-replicates in the transformed cells of the host plant following transactivation.

17. The method of claim 12, wherein the blocking sequence comprises a stuffer sequence flanked on each side by an FRT site in a 5' to 3' orientation.

18. The method of claim 12, wherein the foreign nucleic acid sequence encodes an antigen.

19. The method of claim 18, wherein the antigen is selected from the group consisting of G protein of Respiratory Syncytial Virus, enterotoxin, rabies virus glycoprotein, rabies virus nucleoprotein, hepatitis B surface antigen, Norwalk virus capsid protein, colorectal cancer antigen and gastrointestinal cancer antigen.

20. The method of claim 12, wherein the said cells have one or more types of foreign nucleic acid sequences capable of encoding biopharmaceuticals.

21. The method of claim 20, wherein the biopharmaceuticals are selected from the group consisting of erythropoietins, interferons, insulins, monoclonal antibodies, blood factors, Colony Stimulating Factors, Growth Hormones, Interleukins, Growth Factors and Vaccines.

22. The method of claim 12, wherein the inactive or silenced foreign nucleic acid sequence is introduced into the host plant using an Agrobacterial DNA construct and in which a viral replicon is placed upstream of a target gene and where the viral replicon is a viral nucleic acid sequence which allows for the extrachromosomal replication of a nucleic acid construct in a host cell expressing the appropriate replication factors.

## Patentansprüche

1. Kit zur Herstellung eines gewünschten Fremdpolypeptids in Zellen einer Wirtspflanze, umfassend:
genetisch transformierte Zellen der Wirtspflanze, in deren nukleärem Genom eine inaktive oder gesilencte Fremdnukleinsäuresequenz eingebracht ist, die für ein gewünschtes Fremdpolypeptid kodiert; und
einen rekombinanten RNA-Virusvektor, der dazu in der Lage ist, die genetisch transformierten Zellen der Wirtspflanze zu infizieren und in diesen für eine Rekombinase zu kodieren, wobei die inaktive oder gesilencte Fremdnukleinsäuresequenz eine blockierende Sequenz zwischen der Fremdnukleinsäuresequenz und dem Promotor (und weiteren regulatorischen Sequenzen) umfasst, um die Expression der Fremdnukleinsäuresequenz zu regulieren, wobei die blockierende Sequenz auf jeder Seite von einer Rekombinase-Zielstelle mit einer definierten 5'- zu 3'-Orientierung flankiert ist.

2. Kit nach Anspruch 1, wobei der rekombinante RNA-Virusvektor eine rekombinante genomische Komponente eines Tabakmosaikvirus, eines Alfalfamosaikvirus, eines Ilarvirus, eines Gurkenmosaikvirus oder eines Closterovirus aufweist.

3. Kit nach Anspruch 1, wobei es sich bei der Wirtspflanze um eine zweikeimblättrige oder eine einkeimblättrige Pflanze handelt.

4. Kit nach Anspruch 1, wobei die Fremdnukleinsäuresequenz eine virale Sequenz umfasst, die sich in den transformierten Zellen der Wirtspflanze nach der Transaktivierung selbst repliziert.

5. Kit nach Anspruch 1, wobei die blockierende Sequenz eine Stuffer-Sequenz umfasst, die auf beiden Seiten von einer FRT-Stelle in einer 5'- zu 3'-Orientierung flankiert ist.

6. Kit nach Anspruch 1, wobei die Fremdnukleinsäuresequenz für ein Antigen kodiert.

7. Kit nach Anspruch 6, wobei das Antigen ausgewählt ist aus der Gruppe bestehend aus dem G-Protein des respiratorischen Synzytialvirus, Enterotoxin, dem Glykoprotein des Tollwutvirus, dem Nukleoprotein des Tollwutvirus, dem Hepatitis B-Oberflächenantigen, dem Kapsidprotein des Norwalk-Virus sowie Kolorektalkarzinom-Antigen und Gastrointestinalkarzinom-Antigen.

8. Kit nach Anspruch 1, wobei die Zellen eine oder mehrere Arten an Fremdnukleinsäuresequenzen aufweisen, die dazu in der Lage sind, für Biopharmazeutika zu kodieren.

9. Kit nach Anspruch 8, wobei die Biopharmazeutika ausgewählt sind aus der Gruppe bestehend aus Erythropoietinen, Interferonen, Insulinen, monoklonalen Antikörpern, Blutfaktoren, koloniestimulierenden Faktoren, Wachstumshormonen, Interleukinen, Wachstumsfaktoren und Impfstoffen.

10. Kit nach Anspruch 1, wobei es sich bei dem rekombinanten RNA-Virusvektor um einen heterologen Virusvektor handelt.

11. Kit nach Anspruch 1, wobei die inaktive oder gesilencte Fremdnukleinsäuresequenz unter Verwendung eines DNA-Konstrukts aus dem Agrobakterium in die Wirtspflanze eingebracht wird und wobei ein virales Replikon stromaufwärts eines Zielgens gelegen ist und wobei es sich bei dem viralen Replikon um eine virale Nukleinsäuresequenz handelt, die die extrachromosomale Replikation eines Nukleinsäurekonstrukts in einer Wirtszelle gestattet, die die geeigneten Replikationsfaktoren exprimiert.

12. Verfahren zur Herstellung eines Fremdpolypeptids in Zellen einer Wirtspflanze umfassend:
(a) das Bereitstellen einer transgenen Pflanze, so dass das nukleäre Genom der Zellen der transgenen Pflanze eine transgene DNA aufweist, die eine inaktive oder gesilencte Fremdnukleinsäuresequenz aufweist, die für ein Fremdpolypeptid kodiert;
(b) das Infizieren der Zellen der transgenen Pflanze mit einem rekombinanten RNA-Virusvektor, so dass sich dieser in der Zelle repliziert und eine Rekombinase transient exprimiert; und
(c) das Kultivieren der Pflanze, wobei die Fremdpolypeptide in den Zellen produziert werden; wobei die inaktive oder gesilencte Fremdnukleinsäuresequenz eine blockierende Sequenz zwischen der Fremdnukleinsäuresequenz und dem Promotor (und weiteren regulatorischen Sequenzen) umfasst, um die Expression der Fremdnukleinsäuresequenz zu regulieren, wobei die blockierende Sequenz auf jeder Seite von einer Rekombinase-Zielstelle mit einer definierten 5'- zu 3'-Orientierung flankiert ist.

13. Verfahren nach Anspruch 12, wobei es sich bei dem rekombinanten RNA-Virusvektor um einen heterologen Virusvektor handelt.

14. Verfahren nach Anspruch 12, wobei es sich bei der Wirtspflanze um eine zweikeimblättrige oder eine einkeimblättrige Pflanze handelt.

15. Verfahren nach Anspruch 12, wobei der rekombinante RNA-Virusvektor eine rekombinante genomische Komponente eines Tabakmosaikvirus, eines Alfalfamosaikvirus, eines Ilarvirus, eines Gurkenmosaikvirus oder eines Closterovirus aufweist.

16. Verfahren nach Anspruch 12, wobei die Fremdnukleinsäuresequenz eine virale Sequenz umfasst, die sich in den transformierten Zellen der Wirtspflanze nach der Transaktivierung selbst repliziert.

17. Verfahren nach Anspruch 12, wobei die blockierende Sequenz eine Stuffer-Sequenz umfasst, die auf beiden Seiten von einer FRT-Stelle in einer 5'- zu 3'-Orientierung flankiert ist.

18. Verfahren nach Anspruch 12, wobei die Fremdnukleinsäuresequenz für ein Antigen kodiert.

19. Verfahren nach Anspruch 18, wobei das Antigen ausgewählt ist aus der Gruppe bestehend aus dem G-Protein des respiratorischen Synzytialvirus, Enterotoxin, dem Glykoprotein des Tollwutvirus, dem Nukleoprotein des Tollwutvirus, dem Hepatitis B-Oberflächenantigen, dem Kapsidprotein des Norwalk-Virus sowie Kolorektalkarzinom-Antigen und Gastrointestinalkarzinom-Antigen.

20. Verfahren nach Anspruch 12, wobei die Zellen eine oder mehrere Arten an Fremdnukleinsäuresequenzen aufweisen, die dazu in der Lage sind, für Biopharmazeutika zu kodieren.

21. Verfahren nach Anspruch 20, wobei die Biopharmazeutika ausgewählt sind aus der Gruppe bestehend aus Erythropoietinen, Interferonen, Insulinen, monoklonalen Antikörpern, Blutfaktoren, koloniestimulierenden Faktoren, Wachstumshormonen, Interleukinen, Wachstumsfaktoren und Impfstoffen.

22. Verfahren nach Anspruch 12, wobei die inaktive oder gesilencte Fremdnukleinsäuresequenz unter Verwendung eines DNA-Konstrukts aus dem Agrobakterium in die Wirtspflanze eingebracht wird und wobei ein virales Replikon stromaufwärts eines Zielgens gelegen ist und wobei es sich bei dem viralen Replikon um eine virale Nukleinsäuresequenz handelt, die die extrachromosomale Replikation eines Nukleinsäurekonstrukts in einer Wirtszelle gestattet, die die geeigneten Replikationsfaktoren exprimiert.

## Revendications

1. Kit pour la production d'un polypeptide d'intérêt étranger dans des cellules d'une plante hôte, comprenant :
des cellules transformées génétiquement de la plante hôte ayant intégré, dans son génome nucléaire, une séquence d'acide nucléique étrangère inactive ou silencieuse codant pour un polypeptide d'intérêt étranger ; et
un vecteur viral d'ARN recombinant capable d'infecter les cellules transformées génétiquement de la plante hôte et codant dans celle-ci pour une recombinase, dans lequel la séquence d'acide nucléique étrangère inactive ou silencieuse comprend une séquence de blocage entre la séquence d'acide nucléique étrangère et le promoteur (et autres séquences régulatrices), pour diriger l'expression de la séquence d'acide nucléique étrangère, la séquence de blocage étant flanquée de chaque côté par un site cible de recombinase avec une orientation définie de 5' à 3'.

2. Kit selon la revendication 1, dans lequel le vecteur viral d'ARN recombinant a un composant génomique recombinant d'un tobamovirus, d'un virus de la mosaïque alfalfa, d'un ilarvirus, d'un cucumovirus ou d'un closterovirus.

3. Kit selon la revendication 1, dans lequel la plante hôte est une plante dicotylédone ou monocotylédone.

4. Kit selon la revendication 1, dans lequel la séquence d'acide nucléique étrangère comprend une séquence virale qui s'auto-réplique dans les cellules transformées de la plante hôte après transactivation.

5. Kit selon la revendication 1, dans lequel la séquence de blocage comprend une séquence intercalée flanquée de chaque côté par un site FRT dans une orientation de 5' à 3'.

6. Kit selon la revendication 1, dans lequel la séquence d'acide nucléique étrangère code pour un antigène.

7. Kit selon la revendication 6, dans lequel l'antigène est choisi dans le groupe comprenant la protéine G du virus respiratoire syncytial, une entérotoxine, la glycoprotéine du virus de la rage, la nucléoprotéine du virus de la rage, l'antigène de surface du virus de l'hépatite B, la protéine de capside du virus Norwalk, l'antigène du cancer colorectal et l'antigène du cancer gastro-intestinal.

8. Kit selon la revendication 1, dans lequel lesdites cellules ont un ou plusieurs types de séquences d'acide nucléique étrangères capables de coder pour des agents biopharmaceutiques.

9. Kit selon la revendication 8, dans lequel les agents biopharmaceutiques sont choisis dans le groupe comprenant des érythropoïétines, des interférons, des insulines, des anticorps monoclonaux, des facteurs sanguins, des facteurs de stimulation de colonie, des hormones de croissance, des interleukines, des facteurs de croissance et des vaccins.

10. Kit selon la revendication 1, dans lequel le vecteur viral RNA recombinant est un vecteur viral hétérologue.

11. Kit selon la revendication 1, dans lequel la séquence d'acide nucléique étrangère inactive ou silencieuse est introduite dans la plante hôte en utilisant une construction d'ADN d'Agrobacterium et dans laquelle un réplicon viral est placé en amont d'un gène cible et le réplicon viral étant une séquence d'acide nucléique viral qui permet la réplication extrachromosomique d'une construction d'acide nucléique dans une cellule hôte exprimant les facteurs de réplication appropriés.

12. Procédé de production d'un polypeptide étranger dans des cellules d'une plante hôte comprenant :
(a) la fourniture d'une plante transgénique de sorte que le génome nucléaire des cellules de la plante transgénique ait un ADN transgénique comprenant une séquence d'acide nucléique étrangère inactive ou silencieuse codant pour un polypeptide étranger ;
(b) l'infection des cellules de plante transgénique avec un vecteur viral d'ARN recombinant de sorte qu'il se réplique et exprime provisoirement dans celles-ci, une recombinase ; et
(c) la culture de ladite plante, lesdits polypeptides étrangers étant produits dans lesdites cellules ; la séquence d'acide nucléique étrangère inactive ou silencieuse comprenant une séquence de blocage entre la séquence d'acide nucléique étrangère et le promoteur (et autres séquences régulatrices), pour diriger l'expression de la séquence d'acide nucléique étrangère, la séquence de blocage étant flanquée de chaque côté par un site cible de la recombinase avec une orientation définie de 5' à 3'.

13. Procédé selon la revendication 12, dans lequel le vecteur viral d'ARN recombinant est un vecteur viral hétérologue.

14. Procédé selon la revendication 12, dans lequel la plante hôte est une plante dicotylédone ou monocotylédone.

15. Procédé selon la revendication 12, dans lequel le vecteur viral d'ARN recombinant a un composant génomique recombinant d'un tobamovirus, d'un virus de la mosaïque alfalfa, d'un ilarvirus, d'un cucumovirus ou d'un closterovirus.

16. Procédé selon la revendication 12, dans lequel la séquence d'acide nucléique étrangère comprend une séquence virale qui s'auto-réplique dans les cellules transformées de la plante hôte après transactivation.

17. Procédé selon la revendication 12, dans lequel la séquence de blocage comprend une séquence intercalée flanquée de chaque côté par un site FRT dans une orientation de 5' à 3'

18. Procédé selon la revendication 12, dans lequel la séquence d'acide nucléique étrangère code pour un antigène.

19. Procédé selon la revendication 18, dans lequel l'antigène est choisi dans le groupe comprenant la protéine G du virus respiratoire syncytial, une entérotoxine, la glycoprotéine du virus de la rage, la nucléoprotéine du virus de la rage, l'antigène de surface du virus de l'hépatite B, la protéine de capside du virus Norwalk, l'antigène du cancer colorectal et l'antigène du cancer gastro-intestinal

20. Procédé selon la revendication 12, dans lequel lesdites cellules ont un ou plusieurs types de séquences d'acide nucléique étrangères capables de coder pour des agents biopharmaceutiques.

21. Procédé selon la revendication 20, dans lequel les agents biopharmaceutiques sont choisis dans le groupe comprenant des érythropoïétines, des interférons, des insulines, des anticorps monoclonaux, des facteurs sanguins, des facteurs de stimulation de colonie, des hormones de croissance, des interleukines, des facteurs de croissance et des vaccins.

22. Procédé selon la revendication 12, dans lequel la séquence d'acide nucléique étrangère inactive ou silencieuse est introduite dans la plante hôte en utilisant une construction d'ADN d'Agrobacterium et dans laquelle un réplicon viral est placé en amont d'un gène cible et le réplicon viral étant une séquence d'acide nucléique viral qui permet la réplication extrachromosomique d'une construction d'acide nucléique dans une cellule hôte exprimant les facteurs de réplication appropriés.
